# EUROPEAN PATENT APPLICATION

(11) **EP 4 095 252 A1**
(43) Date of publication of application: **30.11.2022**
(21) Application number: 21175851.1
(22) Date of filing: 26.05.2021
(51) Int. Cl.: C12P 19/30, A23L 33/145, A23L 27/23, C12N 9/16

(54) **PROCESS FOR THE PRODUCTION OF A COMPOSITION CONTAINING 5' -RIBONUCLEOTIDES**

(71) Applicant: DSM IP Assets B.V., 6411 TE Heerlen (NL)
(72) Inventor: VAN BEELEN, Petrus Norbertus, 6100 AA Echt (NL); VAN DEN BERG, Marco Alexander, 6100 AA Echt (NL)
(74) Representative: DSM Intellectual Property

(57) **Abstract**

The present invention describes a to produce a composition containing 5'-ribonucleotides comprising: subjecting a microorganism to autolysis under conditions at which a substantial part of the RNA remains in a form degradable into 5'-ribonucleotides and at which a substantial part of the RNA remains associated with the cell wall fraction; subjecting the autolysate to solid/liquid separation and recovering the RNA-containing cell wall fraction; converting the RNA in the recovered RNA-containing cell wall fraction into 5'-ribonucleotides using a 5'-phosphodiesterase comprising the amino acid sequence according to SEQ ID NO 1, or an amino acid sequence having at least 90% sequence identity to SEQ ID NO 1 or a 5'-phosphodiesterase derived from *Leptographium procerum.*

## Description

### Field of the invention

The present invention relates to a process to produce a composition containing 5'-ribonucleotides. The invention further relates to a compositions containing 5'-ribonucleotides and to the use of said compositions in food or feed.

### Background of the invention

Autolytic yeast extracts are concentrates of the soluble materials obtained from yeast after disruption of the cells and digestion (lysis) of the polymeric yeast material. The active yeast enzymes released in the medium after cell disruption contribute to the lysis. These types of yeast extract are rich in amino acids and generally do not comprise 5'-ribonucleotides because during the autolytic process the native RNA is decomposed or modified in a form which is not degradable into 5'-ribonucleotides. They are used in the food industry as basic taste providers. The amino acids present in the yeast extract add a bouillon-type brothy taste to the food.

Hydrolytic yeast extracts, on the other hand, are concentrates of the soluble materials obtained from yeast after disruption of the cells, digestion (lysis) and addition of proteases and/or peptidases and especially nucleases to the yeast suspension during lysis. The native yeast enzymes can be fully or partially be inactivated prior to the lysis; the latter variation still leading to an yeast autolysate. During this process, 5'-ribonucleotides of guanine (5'-guanine mono phosphate; 5'-GMP), uracil (5'-uracil mono phosphate; 5'-UMP), cytosine (5'-cytosine mono phosphate; 5'-CMP) and adenine (5'-adenine mono phosphate; 5'-AMP) are formed. When adenylic deaminase is added to the mixture, 5'-AMP is transformed into 5'-inosine mono phosphate (5'-IMP). The hydrolytic yeast extracts obtained by this method are therefore rich in 5'-ribonucleotides, especially rich in 5'-GMP and 5'-IMP. Often yeast extracts are also rich in glutamate. 5'-IMP, 5'-GMP and glutamate are known for their flavour enhancing properties. They are capable of enhancing the savoury and delicious taste in certain types of food. This phenomenon is described as 'mouthfeel' or umami.

Yeast extracts rich in 5'-ribonucleotides and, optionally, rich in glutamate, are usually added to soups, sauces, marinades and flavour seasonings.

Yeast extracts rich in 5'-ribonucleotides are up to date produced using yeast strains with high RNA content and/or by partial extraction of the cell content.

A disadvantage of this type of taste enhancing yeast extracts is that, due to the presence of amino acids and short peptides and of others yeast components, they are not very suitable for applications which require a clean taste.

US Patent No. 4,303,680 describes the production of a yeast extract containing 5'-ribonucleotides using an autolytic process under conditions at which the intracellular RNA is only partially decomposed and remains bound to the autolysed cells. The protein content of the cells is hydrolysed in oligopeptides and amino acids. The RNA is enzymatically transformed into 5'-ribonucleotides only after the RNA has been released in solution from the autolysed cells by means of a heat treatment. With this method a yeast extract is obtained which is rich in amino acids, oligopeptides and other components like carbohydrates, minerals, lipids and vitamins. The presence of these components imparts to this yeast extract a bouillon-like, brothy taste which is not desirable in some food applications. A further disadvantage is that the yeast extract comprises a relatively low amount of 5'-ribonucleotides.

WO2005067734 discloses a process for the production of a composition containing 5'-ribonucleotides which is based on autolysis of a microorganism. Specifically disclosed is a method comprising subjecting a microorganism to autolysis under conditions at which a substantial part of the RNA remains in a form degradable into 5'-ribonucleotides and at which a substantial part of the RNA remains associated with the cell wall fraction; subjecting the autolysate to solid/liquid separation and recovering the RNA-containing cell wall fraction; and converting the RNA in the recovered RNA-containing cell wall fraction into 5'-ribonucleotides. The disadvantage of this process is a suboptimal conversion of RNA into 5'-ribonucleotides, presumably because enzymatic treatment of RNA that remains associated with the cell well is not optimal.

Therefore, there is a need in the art for further improved processes for the production of a composition containing 5'-ribonucleotides.

### Detailed description of the invention

In a first aspect the present invention provides a process to produce a composition containing 5'-ribonucleotides comprising:
a) subjecting a microorganism to autolysis under conditions at which a substantial part of the RNA remains in a form degradable into 5'-ribonucleotides and at which a substantial part of the RNA remains associated with the cell wall fraction;
b) subjecting the autolysate to solid/liquid separation and recovering the RNA-containing cell wall fraction;
c) converting the RNA in the recovered RNA-containing cell wall fraction into 5'-ribonucleotides using a 5'-phosphodiesterase comprising the amino acid sequence according to SEQ ID NO 1, or an amino acid sequence having at least 90% sequence identity to SEQ ID NO 1 and/or a 5'-phosphodiesterase derived from *Leptographium procerum.*

Surprisingly, the present inventors found that a 5'-phosphodiesterase comprising the amino acid sequence according to SEQ ID NO 1 and/or derived from *Leptographium procerum* provides a higher yield of 5'-ribonucleotides than enzymes having similar functionalities.

With the term "5'-ribonucleotides" it is herewith intended a mixture of 5'-GMP, 5'-CMP, 5'-UMP and further 5'-AMP and/or 5'-IMP, wherein said 5'-AMP may be either partially or completely converted into 5'-IMP.

The term "5'-ribonucleotide(s)" encompasses the free 5'-ribonucleotide as well as a salt thereof.

In the context of the present invention autolysis of a microorganism is defined as a process wherein degradation of the microbial cells and of the polymeric microbial material is at least partially effected by active native microbial enzymes released in the medium after (partially) damaging and/or disrupting the microbial cell wall.

Any microorganism can be used as natural source of RNA in the process of the invention. Bacterial and fungal microorganisms are preferred, such as those which are suitable for food and feed applications. Preferred microorganisms are those that have the status of being food-grade and that can be safely applied in food for human consumption. Bacterial or fungal strains with a high RNA content (i.e. with an RNA content of typically 6-15%) enable the production of compositions with a high amount of 5'-ribonucleotides. However an advantage of the process of the invention is that also bacterial or fungal strains with a relatively low RNA content can be used. These strains can be advantageously used for the preparation of compositions containing a higher 5'-ribonucleotide content than would be expected on basis of the RNA content of the starting strain.

Examples of preferred microorganisms include filamentous fungi such as *Trichoderma, Fusarium* or *Aspergillus,* and yeasts such as *Saccharomyces, Torula, Pichia, Yarrowia, Kluyveromyces* and *Candida.* Strains belonging to the genus *Saccharomyces,* in particular belonging to the species *Saccharomyces cerevisiae* are most preferred.

Examples of suitable bacterial microorganisms are lactic acid bacteria, e.g. *Lactobacillus.*

The microorganism used in the process of the invention may be prepared by any suitable fermentation process known in the art. The microbial biomass may be concentrated prior to its use in the present process, for example by centrifugation or filtration. For example, cream yeast (baker's yeast which has been concentrated to 15-27% w/w) may be used. Optionally fermentation broths comprising Brewer's yeast or residue yeast derived from breweries (spent Brewer's yeast) may be used.

The present 5'-phosphodiesterase can be produced by fermentation of the fungus *Leptographium procerum.* Details on the enzyme and production process are disclosed in *Safety evaluation of phosphodiesterase derived from Leptographium* procerum, Steensma et al, Food and Chemical Toxicology 42 (2004) 935-944). The enzyme is commercially available from DSM.

Preferably, the present 5'-phosphodiesterase comprises an amino acid sequence having at least 91, 92, 93, 94, 95, 96, 97, 98 or 99% sequence identity to SEQ ID NO 1.

The present invention provides a process which is especially suitable for large scale production of compositions containing 5'-ribonucleotides. Large scale means that fermentation is performed in fermentors of more than 10 m³.

The autolytic process is initiated by damaging and/or partially disrupting the microbial cell walls. This way the cells are partially opened and at least some of the cell content is released. In order to damage and/or partially disrupt the microbial cell walls, the cells are treated chemically, mechanically or enzymatically using methods known to those skilled in the art.

Mechanical treatments include homogenisation techniques. At this purpose, use of high-pressure homogenisers is possible. Other homogenisation techniques may include mixing with particles, e.g. sand and/or glass beads, or the use of a milling apparatus (e.g. a bead mill).

Chemical treatments include the use of salts, alkali and/or one or more surfactants or detergents. Chemical treatments are less preferred because they may lead to partial degradation of RNA especially when alkali is used, with consequent formation of 2'-ribonucleotides and 3'-ribonucleotides.

Preferably damaging and/or partially disrupting the microbial cell wall is done enzymatically because a better control of the process can thereby be achieved and because this method is especially suitable to be used at large scale. Several enzyme preparations can be used like cellulases, glucanases, hemicellulases, chitinases, proteases and/or pectinases. Preferably protease is used, more preferably endoprotease is used. The conditions used to initiate the autolytic process are dependent on the type of enzyme used and can be easily determined by those skilled in the art. Generally the conditions used to enzymatically damage and/or disrupt the microbial cell wall will correspond to those applied during the autolysis of the microorganism.

The autolysis of the microorganism is at least partially effected by active native microbial enzymes released in solution after (partially) damaging and/or disrupting the microbial cell wall wherein the chemicals, or more preferably, the enzymes added to damage and/or to disrupt the microbial cell wall may contribute to the degradation of the microbial cells and of polymeric microbial material.

In the process of the invention the conditions used in the autolytic process are such that a substantial part of the RNA remains in a form degradable into 5'-ribonucleotides. In this context, with "substantial part of the RNA" is meant preferably at least 50%, more preferably at least 60%, most preferably at least 70%. Thus, the RNA does not need to remain fully intact during the autolytic process, but at least a substantial part of the RNA should remain in a form degradable into 5'-ribonucleotides. Generally up to 100% of the RNA may remain in a form degradable into 5'-ribonucleotides. In a form degradable into 5'-ribonucleotides means that the RNA should be in a form that allows conversion into 5'-ribonucleotides by a suitable enzyme. Preferably the suitable enzyme is a 5'-phosphodiesterase (5'-Fdase).

A form of RNA degradable into 5'-ribonucleotides comprises oligonucleotides containing at least two ribonucleotide units. Therefore RNA in a form degradable into 5'-ribonucleotides may consist of a mixture comprising intact RNA and oligonucleotides or polynucleotides of different lengths. In the context of the present invention an oligonucleotide comprises 2-10 ribonucleotide units, while a polynucleotide comprises more than 10 ribonucleotide units.

In the process of the invention the conditions used in the autolytic process are such that during the autolysis a substantial part of the RNA remains associated with the cell wall fraction, i.e. remains inside the damaged cells and/or bound to the cell walls or fragments thereof. In this context, with "substantial part of the RNA" is meant preferably at least 20%, more preferably at least 30%, most preferably at least 40%. Generally up to 90% of the RNA may remain associated with the cell wall fraction. Preferably, up to 100% of the RNA remains associated with the cell wall fraction.

The percentage of RNA, which remains in a form degradable into 5'-ribonucleotides during the autolytic process is defined as the ratio (x 100) between a) the weight percentage of 5'-GMP (calculated as the disodium heptahydrate thereof and based on sodium chloride free dry matter) measured in the autolysate after inactivation of the enzymes participating in the autolysis and conversion of RNA into 5'-ribonucleotides, and b) the weight percentage of GMP (calculated as the disodium heptahydrate thereof and based on sodium chloride free dry matter) measured in the starting material after complete alkaline hydrolysis of RNA. The weight percentage of GMP (calculated as the disodium heptahydrate thereof and based on sodium chloride free dry matter) measured in the starting material after alkaline hydrolysis can be determined from the corresponding weight percentage of free GMP (based on sodium chloride free dry matter) by multiplying the latter with a factor 1.47.

The percentage of the RNA, which remains associated with the cell wall fraction is defined as the ratio (x100) between a) the amount of RNA in grams in the cell wall fraction of an autolysate originating from a fixed amount of starting material, and b) the amount of RNA in grams present in the same fixed amount of starting material.

The conditions applied in the autolysis to ensure that a substantial part of the RNA remains in a form degradable into 5-ribonucleotides and that a substantial part of the RNA remains associated with the cell wall fraction, will be generally dependent on the microorganism used. These conditions can be easily determined by those skilled in the art by varying process parameters like temperature and/or pH and/or the time period at which a particular temperature and/or pH is maintained during autolysis and subsequently determining the effect of such process parameter(s) on the amount of RNA which remains in a form degradable into 5-ribonucleotides and/or which remains associated with the cell wall fraction.

In particular the first phase of autolysis is performed at a particular pH range combined with a particular temperature.

For instance, the conditions applied in the autolysis of *Saccharomyces cerevisiae* to ensure that a substantial part of the RNA remains in a form degradable into 5'-ribonucleotides and that a substantial part of the RNA remains associated with the cell wall fraction are such that the pH in the first phase of the autolysis is between 4.5-9 and/or the temperature is between 50-65°C. Preferably the first 8 hours of the autolysis, more preferably the first 4 hours of the autolysis, are performed at a pH of 4.5-5.5 and at a temperature of 57-65°C, or at a pH 5.5-9 and a temperature of 50-65°C.

The conditions to be kept after the first phase of the autolysis are less critical. After the first phase the pH is generally kept between 4 and 10 and the temperature is generally kept between 40°C and 70°C. In general the duration of the autolytic process including the first phase is at most 24 hours.

The present invention may encompass as well a process wherein in step a) a microorganism is subjected to hydrolysis under conditions at which a substantial part of the RNA remains in a form degradable into 5'-ribonucleotides and at which a substantial part of the RNA remains associated with the cell wall fraction. In the context of the present invention hydrolysis of a microorganism is defined as a process wherein the native microbial enzymes have been partly inactivated and wherein suitable exogenous enzymes added to the microbial biomass to effect degradation of the microbial cells and of the polymeric microbial material.

After autolysis a suspension (autolysate) is obtained which comprises a microbial cell wall fraction, RNA which is for a substantial part in a form degradable into 5'-ribonucleotides and which is for a substantial part associated with the cell wall fraction, and soluble cell components (e.g. proteins, carbohydrates, etcetera). The cell wall fraction comprises insoluble cell residues, in particular cell walls or fragments thereof.

At the end of the autolytic process and prior to step b), the chemicals used for damaging and/or partially disrupting the microbial cell walls and/or the enzymes which took part in the autolytic process should preferably be neutralised and/or inactivated. The enzymes which took part in the autolysis, are the native microbial enzymes and optionally any added exogenous enzyme used to initiate the autolytic process. Neutralisation and/or inactivation of the chemicals and/or the enzymes should occur under conditions at which a substantial part of the RNA remains in a form degradable into 5'-ribonucleotides and wherein a substantial part of the RNA remains associated with the cell wall fraction. Inactivation of the enzymes which took part in the autolysis can be done by pH treatment or preferably by a heat treatment whereby the enzymes are inactivated, a substantial part of the RNA remains in a form degradable into 5'-ribonucleotides and wherein a substantial part of the RNA remains associated with the cell wall fraction. The enzymes can be inactivated by heat treatment, for instance by heating the mixture from 5 minutes to 1 hour at a temperature from 65°C to 95°C, more preferably by heating from 30 minutes to 1 hour at a temperature from 65°C to 75°C, wherein typically a shorter reaction time may be used at higher reaction temperatures. For example, heating the mixture for 1 hour at 65°C, or for 30 minutes at 75°C may be sufficient to inactivate the enzymes whereby a substantial part of the RNA remains in a form degradable into 5'-ribonucleotides and wherein a substantial part of the RNA remains associated with the cell wall fraction.

In step b) of the process of the invention the autolysate is subjected to solid/liquid separation and the RNA-containing cell wall fraction is recovered.

The RNA-containing cell wall fraction is preferably recovered by common solid-liquid separation methods, preferably by centrifugation or filtration. Use of centrifugation or filtration is economically advantageous in particular when the process is performed at large scale.

In order to increase the amount of recovered RNA in a form degradable into 5'-ribonucleotides, the autolysate may be subjected to ultra filtration (UF) instead of to a common solid-liquid separation method like centrifugation or filtration. In this way, a mixture of the RNA-containing cell wall fraction and RNA derived from the microbial soluble fraction is recovered. Thus, not only the RNA associated with the cell wall fraction is separated from the microbial soluble fraction but also the RNA which had been released into solution during autolysis. In cases where UF is used to recover RNA, preferably membranes with a molecular weight cut off from 10 to 50 kD or preferably from 20 to 50 kD can be used. In general a larger molecular weight cut off allows a higher flow rate through the membrane, but might result in larger losses and/or less pure products. The type of solid-liquid separation used and the efficiency of said solid-liquid separation can influence, among other factors, the amount of 5'-ribonucleotides obtained in the compositions of the invention.

Alternatively, first the RNA-containing cell wall fraction is preferably recovered by common solid-liquid separation methods, like centrifugation or filtration, and subsequently the supernatant is further subjected to ultra filtration (UF), resulting in two RNA-containing fractions: the RNA-containing cell wall fraction and the RNA-containing UF-fraction.

The RNA in the recovered fraction is converted into 5'-ribonucleotides. This is done by enzymatically treating the RNA-containing cell wall fraction, optionally mixed with RNA derived from the microbial soluble fraction obtained by UF.

5'-phosphodiesterase (5'-Fdase) is preferably used to convert RNA into 5'-ribonucleotides. 5'-phosphodiesterase can be obtained from a microbial or a vegetable source (for example a malt root extract). An example of a commercially available microbial 5'-Fdase is Enzyme RP-1 produced by Amano (Japan), also known as NP1 or Nuclease P1.

Optionally, 5'-AMP is converted to 5'-IMP by a deaminase, for example adenyl deaminase. An example of a commercially available deaminase is Deaminase 500 produced by Amano (Japan).

Treatment of RNA by 5'-Fdase and deaminase can be performed in a two-step or in a single step process.

After conversion of the RNA into 5'-ribonucleotides the fraction containing 5'-ribonucleotides preferably is separated from the cell wall fraction. Said separation may be achieved by centrifugation or filtration or by any other method suitable to achieve solid/liquid separation.

In an embodiment, the fraction containing 5'-ribonucleotides is purified from components having a higher molecular weight than the 5'-ribonucleotides by ultrafiltration. The degree of purification will depend on the molecular weight cut-off of the ultrafiltration membrane used. Ultra filtration membranes can e.g. be used.

It will be understood that in the context of the present invention a wording like "recovering the RNA" or "converting the RNA" does not necessarily mean that all RNA is recovered or converted, respectively. It will be clear to those skilled in the art that the amount of the RNA which is recovered will depend on the type of separation method used. It will also be clear that the amount of RNA which is converted will depend on several factors, one of which is the accessibility of the RNA associated with the cell wall insoluble fraction to the enzymes used in this step.

The present invention provides a process which is especially useful for large-scale separation of RNA and which allows the production of compositions containing 5'-ribonucleotides on a commercially attractive and large scale for use in food and feed applications. The present invention provides a process, which results in a good purity of the composition containing 5'-ribonucleotides and rather low losses.

The invention will now be illustrated by an example which however do not intend to be limiting.

SEQ ID NO 1 refers to 5'-phosphodiesterase derived from *Leptographium procerum.*

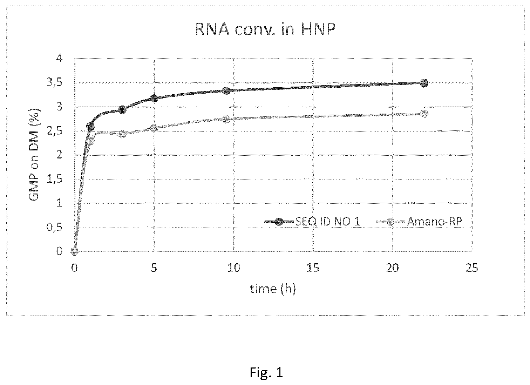

Figure 1 shows the amount of 5'-GMP over time.

### Example 1

### Preparation of a composition comprising 5'-ribonucleotides using different 5'-phosphodiesterases.

2 liter of cream yeast from *Saccharomyces cerevisiae* was warmed up to 60 °C. Subsequently 0.4 ml Alcalase (commercially available protease from Novozymes) was added and the mixture was incubated for 4 hours at pH 6.0 and 60 °C. The conditions were adjusted to pH 5.1 and 51.5 °C and an additional 2 ml of Alcalase was added to the reaction mixture. The mixture was incubated for 20 hours at pH 5.1, 51.5 °C. Next, the mixture or autolysate was heated for 1 hour at 65 °C to inactivate all enzyme activity. The extract (soluble fraction) was separated from the insoluble cell walls by means of centrifugation.

The resulting cell wall fraction was treated with 5'-phosphodiesterase to hydrolyse the RNA into 5'-ribonucleotides at a temperature of 65°C and a pH of 5.3. One 5'-phosphodiesterase was derived from *Leptographium procerum* (according to the invention) and is commercially available from DSM and the other 5'-phosphodiesterase was RP-1 that is commercially available from Amano, Japan (comparative, not according to the invention). Both enzymes were dosed in 5950 U/kg dry matter. During the incubation at 64°C samples were taken during the incubation. All samples were directly heat shocked to allow to stop the enzymatic conversion. All samples were analysed for 5'-GMP. The results are shown in Figure 1 and table 1 below.

| Time (in hours) | Amount 5'-GMP (w/w on dry matter) with SEQ ID NO 1 | Amount 5'-GMP (w/w on dry matter) with RP-1 |
|---|---|---|
| 1 | 2.59 | 2.29 |
| 3 | 2.94 | 2.43 |
| 5 | 3.17 | 2.55 |
| 9.5 | 3.33 | 2.74 |
| 22 | 3.49 | 2.85 |

The results clearly show that using the enzyme derived from *Leptographium procerum*

## Claims

1. Process to produce a composition containing 5'-ribonucleotides comprising:
a) subjecting a microorganism to autolysis under conditions at which a substantial part of the RNA remains in a form degradable into 5'-ribonucleotides and at which a substantial part of the RNA remains associated with the cell wall fraction;
b) subjecting the autolysate to solid/liquid separation and recovering the RNA-containing cell wall fraction;
c) converting the RNA in the recovered RNA-containing cell wall fraction into 5'-ribonucleotides using a 5'-phosphodiesterase comprising the amino acid sequence according to SEQ ID NO 1, or an amino acid sequence having at least 90% sequence identity to SEQ ID NO 1 or a 5'-phosphodiesterase derived from *Leptographium procerum.*

2. Process according to claim 1, comprising:
d) separating the fraction containing 5'-ribonucleotides from the cell wall fraction.

3. Process according to claim 1 or 2, wherein autolysis in a) is initiated by damaging and/or partially disrupting the microbial cell walls.

4. Process according to claim 3, wherein damaging and/or partially disrupting the microbial cell walls is performed enzymatically.

5. Process according to any one of claims 1 to 4 wherein in a) at least 50% of the RNA remains in a form degradable into 5'-ribonucleotides, more preferably at least 60%, most preferably at least 70%.

6. Process according to any one of claims 1 to 5, wherein in a) at least 20% of the RNA remains associated with the cell wall fraction, preferably at least 30%, most preferably at least 40%.

7. Process according to any one of claims 1 to 6, wherein in b) the RNA-containing cell wall fraction is recovered by centrifugation or filtration.

8. Process according to any one of claims 1 to 6, wherein in b) the autolysate is subjected to ultrafiltration whereby a mixture of RNA-containing cell wall fraction and RNA derived from the microbial soluble fraction is recovered.

9. Process according to claim 8, wherein in c) the RNA in the recovered mixture of RNA-containing cell wall fraction and recovered RNA derived from the microbial soluble fraction are converted into 5'-ribonucleotides.

10. Process according to any one of claims 1 to 9, wherein in c) the RNA is also enzymatically converted into 5'-ribonucleotides by deaminase.
